# EUROPEAN PATENT SPECIFICATION

(11) **EP 4 110 419 B1**
(45) Date of publication and mention of the grant of the patent: **23.07.2025**
(21) Application number: 21712656.4
(22) Date of filing: 24.02.2021
(51) Int. Cl.: A61M 1/00, A61F 13/05

(54) **ENDOLUMINAL SEAL AND METHODS OF USE**
ENDOLUMINALE DICHTUNG UND VERWENDUNGSVERFAHREN
ÉLÉMENT D'ÉTANCHÉITÉ ENDOLUMINAL ET PROCÉDÉS D'UTILISATION

(30) Priority: 26.02.2020 US 202062981745 P
(43) Date of publication of application: 04.01.2023
(73) Proprietor: Boston Scientific Scimed, Inc., Maple Grove, MN 55311 (US)
(72) Inventor: MURRAY, Collin, Maynard, MA 01754 (US); KING, Joseph W., Franklin, MA 02038 (US); PIC, Andrew, Northboro, MA 01532 (US); LYDECKER, Lauren, Millbury, MA 01527 (US); RYAN, Shawn, Littleton, MA 01460 (US)
(74) Representative: Vossius & Partner Patentanwälte Rechtsanwälte mbB
(86) International application number: PCT/US2021/019363
(87) International publication number: WO 2021/173631

(56) References cited:
- DE-A1- 102014 005 679
- US-A1- 2014 031 773

## Description

### CROSS-REFERENCE TO RELATED APPLICATIONS

This application claims the benefit of priority from U.S. Provisional Application No. 62/981,745, filed on February 26, 2020.

### TECHNICAL FIELD

This disclosure relates to minimally invasive (e.g., endoscopic and/or laparoscopic) medical devices and related methods of use. In embodiments, the disclosure relates to one or more devices for sealing perforations, leaks, or wounds in the gastrointestinal tract, devices for advancing these sealing devices, and related methods of use, among other aspects. An example of a prior art device is disclosed in US 2014/0031773 A1.

### BACKGROUND

Endoscopic and open surgical procedures of the gastrointestinal (Gl) tract include, for example, colonic resection, bariatric surgery, esophagectomy, gastric bypass, and sleeve gastrectomy, among others. These procedures may result in perforation, post-surgical leaks, or other wounds of the tract. Limited treatment options exist for managing such wounds, which have significant morbidity and mortality rates. Options include surgical re-operation and endoscopic placement of a stent or clips. Surgery is relatively invasive and also has high morbidity and mortality rates. Endoscopic stent placement is a less invasive option. The placed stent, however, can migrate from the intended location and/or wall off infection at the treatment site, inhibiting drainage.

### SUMMARY OF THE DISCLOSURE

According to an aspect, a medical system includes a porous body connected to a distal end of a vacuum tube, and a patch defining a lumen therethrough for accommodating the vacuum tube, wherein the patch is configured to fluidly seal the porous body from a body lumen when the patch is deployed in a subject.

A diameter of the lumen of the patch may be approximately equal to an outer diameter of the vacuum tube, and the patch may be slidable along an exterior of the vacuum tube.

The patch may include a patch tube extending from a first surface of the patch, wherein the patch tube may define a tube lumen in fluid communication with the lumen of the patch, and wherein the patch tube may be configured to seal around a circumference of the vacuum tube when the patch is deployed in the subject.

The patch may include Chitosan.

The medical system may further comprise a sealing fluid configured to be applied to an intersection between the patch and a tissue of the subject.

A diameter of the lumen of the patch may be greater than an outer diameter of the vacuum tube, and wherein a gap may be formed between the patch and the vacuum tube when the patch and the vacuum tube are deployed in the body.

A sealing fluid may be configured to be supplied to the gap to seal the body lumen from the porous body.

The patch may define a plurality of recesses on a surface of the patch facing the porous body, and wherein at least one recess of the plurality of recesses may be configured to overlap a tissue of the body lumen in the deployed configuration.

The plurality of recesses may be in fluid communication with a lumen of the vacuum tube.

The plurality of recesses may communicate with a lumen of the vacuum tube via one or more channels in the patch.

The porous body may include a sponge.

The medical system further comprises an overtube configured to contact the patch during a deployment of the patch.

The overtube is configured to be releasably attached to the patch during the deployment of the patch, and is configured to be disconnected from the patch when the patch is deployed in the subject.

The porous body may include a plurality of holes, pores, or channels which may permit fluid outside of the porous body to flow through the porous body and into a lumen of the vacuum tube.

The medical system may further comprise a vacuum device configured to be attached to a proximal end of the vacuum tube and may be configured to supply a negative pressure to the porous body.

According to another aspect, a medical system comprises a flexible tube having a central vacuum lumen, a porous body attached to a distal end of the flexible tube, and a patch extending radially outward from the flexible tube, wherein the patch includes a plurality of recesses in fluid communication with the central lumen.

The porous body may configured to be placed at a target site in a subject, and wherein a negative pressure may be supplied to the central vacuum lumen and may be configured to cause the patch to adhere to a tissue of a body lumen of the subject via the plurality of recesses.

A fluid disposed at the target site may be configured to pass through holes, pores, or channels in the porous body and at least one recess of the plurality of recesses in the patch toward a proximal end of the flexible tube.

According to yet another aspect, not forming part of the claimed invention, there is disclosed a method of treating a target site of a body lumen of a subject comprises advancing a porous body to the target site, wherein the porous body is attached to a distal end of a flexible tube defining a central lumen, advancing a patch over the flexible tube, connecting the patch to a tissue of the body lumen, and removing a fluid from the target site when the patch is connected to the tissue.

The method may further comprise supplying a negative pressure to the central lumen, wherein the negative pressure is configured to one or more of remove the fluid from the target site or connect the patch to the tissue of the body lumen.

### BRIEF DESCRIPTION OF THE DRAWINGS

The accompanying drawings, which are incorporated in and constitute a part of this specification, illustrate various exemplary embodiments and together with the description, serve to explain the principles of the disclosed embodiments.
FIG. 1 is a schematic view of an endoluminal vacuum therapy (EVAC) system according to an embodiment;
FIGS. 2 and 3 are schematic view of the EVAC system of FIG. 1 deployed in a body, according to an embodiment;
FIG. 4 is a schematic view of another EVAC system deployed in the body, according to an embodiment;
FIG. 5A is a schematic view of another EVAC system deployed in the body, according to an embodiment;
FIG. 5B is a schematic view of another EVAC system deployed in the body, according to an embodiment;
FIG. 5C is a schematic view of another EVAC system deployed in the body, according to an embodiment; and
FIG. 5D is a cross-section of a patch of the EVAC system of FIG. 5C, according to an embodiment.

### DETAILED DESCRIPTION

For ease of description, portions of the disclosed devices and/or their components are referred to as proximal and distal portions. It should be noted that the term "proximal" is intended to refer to portions closer to a user of the devices, and the term "distal" is used herein to refer to portions further away from the user. Similarly, "extends distally" indicates that a component extends in a distal direction, and "extends proximally" indicates that a component extends in a proximal direction. Further, as used herein, the terms "about," "approximately," and "substantially" indicate a range of values within +/- 10% of a stated or implied value. Terms that indicate the geometric shape of a component/surface refer to exact and approximate shapes. This disclosure may be understood with reference to the following description and the appended drawings, wherein like elements are referred to with the same reference numerals.

Endoluminal vacuum therapy (EVAC) has been proposed. In EVAC, negative pressure is delivered to the wound site in the GI tract, for example through a nasogastric tube having a sponge at its terminal end. The sponge is placed endoscopically into the perforation, leak, or other wound. Negative pressure then is applied. Devices and systems suited for EVAC are limited, however.

Embodiments of this disclosure include devices, systems, and methods for endoluminal vacuum therapy (EVAC). In examples, EVAC includes endoluminal placement of a sponge or other like material into the wound site (e.g., a target site), including a perforation, a leak, a cyst, an anastomosis, etc. Placement of the material may be via a catheter, scope (endoscope, bronchoscope, colonoscope, duodenoscope, gastroscope, etc.), tube, or sheath, inserted into the GI tract via a natural orifice. The orifice can be, for example, the nose, mouth, or anus, and the placement can be in any portion of the GI tract, including the esophagus, stomach, duodenum, large intestine, or small intestine. Placement of the material also can be in other organs (e.g., pancreas) reachable via the GI tract.

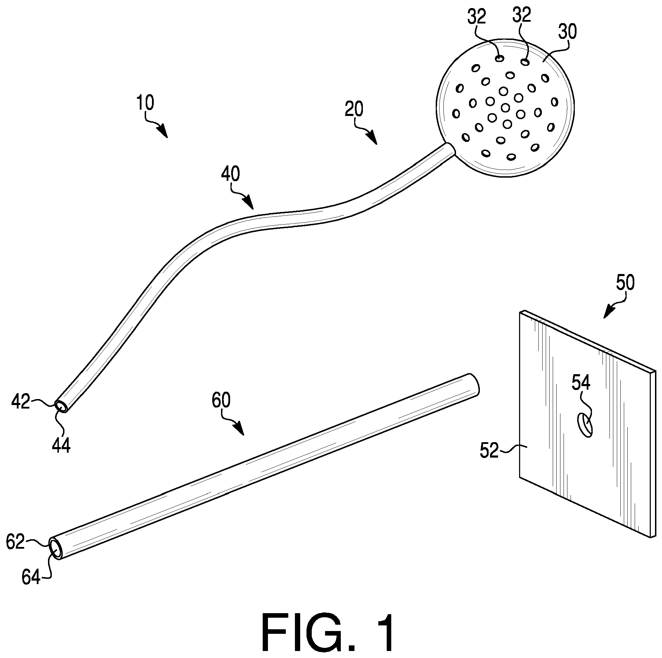

FIG. 1 illustrates a distal end of an EVAC system 10 in accordance with an example of this disclosure. System 10 may be inserted into a patient for treatment of chronic wounds using negative pressure via a vacuum. System 10 generally includes an EVAC device 20, a patch 50, and an overtube 60.

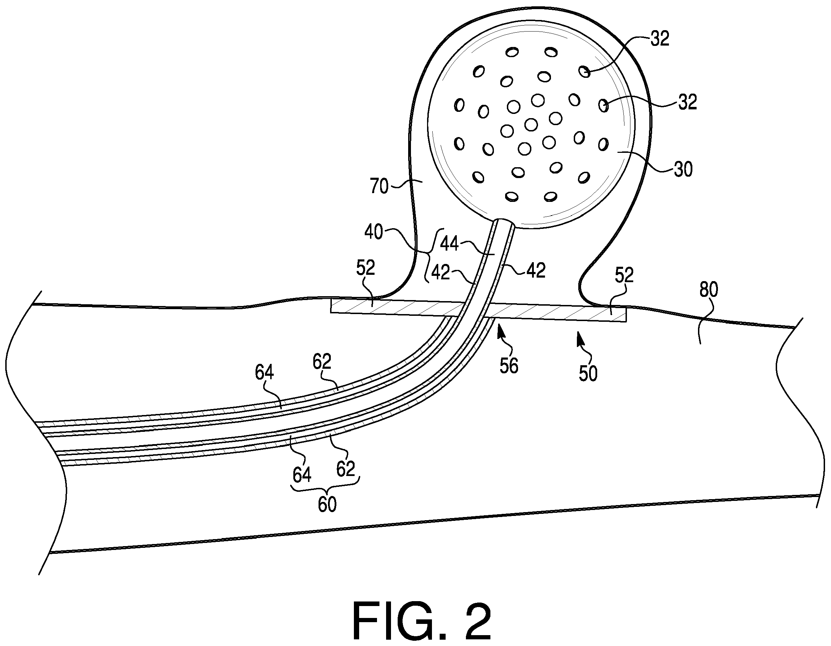

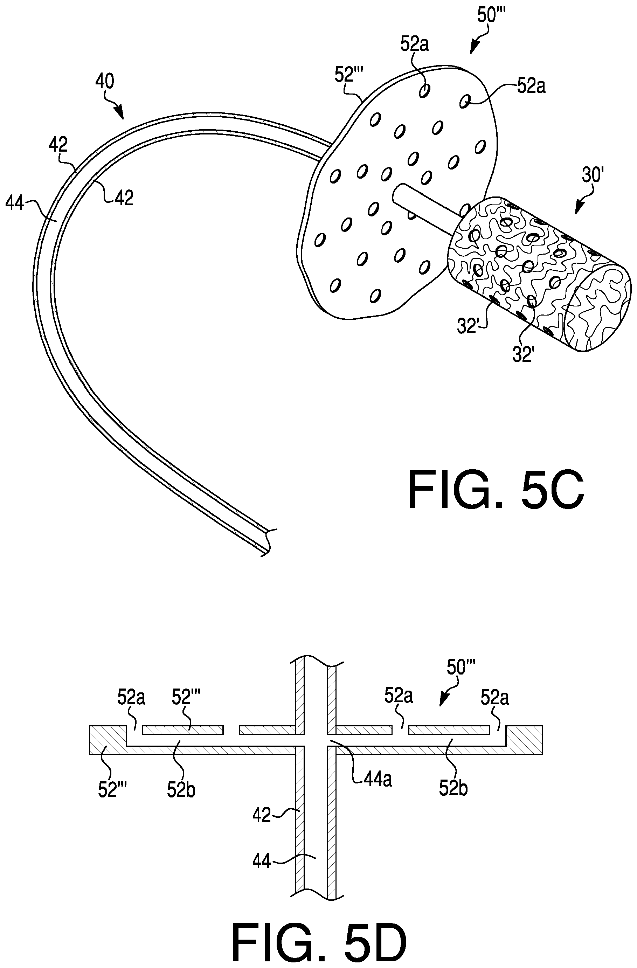

EVAC device 20 may include a sponge 30 (or other mesh-like material or porous body) and a vacuum tube 40. Sponge 30 is attached to a distal end of vacuum tube 40. Sponge 30 may include openings 32 on an outer surface thereof. Openings 32 may be any hole, pore, or channel which provides access to interconnecting channels and pores throughout sponge 30. Openings 32 may include different sizes and shapes, and may be selected based on a location of treatment within the body. Sponge 30 is illustrated as having a spherical shape, but may be any shape, including cylindrical (see, e.g., FIG. 5C), cuboidal, irregular, or the like. Sponge 30 may be compressed into a lower profile during insertion to a target site and may expand during deployment at a target site 70 (see, e.g., FIG. 2).

In embodiments of this disclosure, sponge 30 may be any suitable biocompatible material that may absorb liquids and/or permit liquid or other materials to pass therethrough via negative pressure. The material may be flexible, compressible, porous, hydrophilic, sterile, and/or disposable. The sponge material may be an open-cell foam. Suitable materials include polyurethanes, esters, ethers, composite materials, and other medical-grade materials.

Vacuum tube 40 may include an outer wall 42 defining one or more lumens 44. Lumen 44 is open at both a proximal end and the distal end of vacuum tube 40. Outer wall 42 may include a plurality of holes around a circumference of the distal end of vacuum tube 40 and in fluid communication with lumen 44, which may increase the flow of fluid or material into lumen 44 as disclosed herein. The distal end of vacuum tube 40 may be attached to sponge 30 via sutures, an adhesive, or the like. In one example, a recess (not shown) may be provided in sponge 30 to receive the distal end of vacuum tube 40. Vacuum tube 40 may be attached within the recess of sponge 30, which may provide additional structural support between sponge 30 and vacuum tube 40. The proximal end of vacuum tube 40 may be connected to a vacuum source (not shown), which may supply a negative pressure to sponge 30. For example, a negative pressure of approximately 125 mm Hg, or approximately 2.5 pounds per square inch (PSI), may be supplied to sponge 30. Other suitable amounts of negative pressure may be used. This negative pressure may pull fluid, material, and/or other debris into lumen 44 of vacuum tube 40 via openings 32, which may promote healing of target site 70 (FIG. 2).

With continued reference to FIG. 1, patch 50 may include a body 52 with a lumen 54 extending from a first side of patch 50 through to a second, opposite side of patch 50. As will be explained herein, vacuum tube 40 may extend through lumen 54. Lumen 54 may be sized and shaped to create a fluid seal with vacuum tube 40. For example, a diameter of lumen 54 may be approximately equal to a diameter of an outer surface of vacuum tube 40, but is not limited thereto. In some embodiments, the diameter of lumen 54 may be smaller than an outer diameter of tube 40. In such an instance, a flexibility of patch 50 may permit tube 40 to fit within lumen 54. Such a tighter fit may provide an even more effective seal. In some instances, patch 50 (and any patch described herein) may be thicker around lumen 54 and/or may include a different or an additional material having different mechanical properties, which may prevent tears in patch 50 at lumen 54.

Patch 50 may be any size and shape sufficient to seal an opening between a GI tract 80 (e.g., a body lumen) and target site 70 (FIG. 2). Patch 50 is shown as rectangular. The shape of patch 50, however, could be circular, irregular, etc. Patch 50 also may be cut to the shape of the opening to target site 70. According to an example, patch 50 may include Chitosan or other similar material that is configured to adhere to tissue of GI tract 80 and create a seal with the tissue. Alternatively, patch 50 may include any medical grade material suitable for sealing the opening between GI tract 80 and target site 70. In embodiments, patch 50 may include a material that becomes an adhesive when activated by a complementary fluid/agent, for example, pH based crosslinking polymers, temperature or light activated polymers that crosslink to tissue, or the like. Patch 50 may be durable and may be suitable for use in GI tract 80 for two to three days, or more, without being replaced. Patch 50 (and any patch describe in this disclosure) may be flexible and/or compressible for delivery through a tortuous body lumen to target site 70. In some instances, lumen 54 may be formed in patch 50 by, e.g., cutting, after patch 50 is delivered to target site 70. In this instance, sponge 30 may be delivered in a compressed state through lumen 54 to target site 70, and sponge 30 may expand from the compressed state to an expanded state at target site 70 in any manner described in this disclosure.

Overtube 60 may include an outer wall 62 defining a central lumen 64, and central lumen 64 may extend from a distal end of overtube 60 to a proximal end thereof. As will be explained herein, overtube 60 may be configured to be placed over vacuum line 40 to push patch 50 into the correct position at target site 70. A diameter of lumen 64 may be greater than the diameter of the outer surface of outer wall 42 of vacuum tube 40, which may allow overtube 60 to be moved in distal and proximal directions relative to vacuum tube 40. Overtube 60 may be made of any medical grade material, and may include a columnar strength suitable to push patch 50 along vacuum line 40 toward sponge 30. According to an example, a handle or other gripping member may be provided at the proximal end of overtube 60, which may assist in moving overtube 60 relative to vacuum line 40. In this manner, the distal end of overtube 60 may push patch 50 along vacuum line 40 to target site 70.

An example of system 10 disposed in the body at target site 70 is shown in FIG. 2. Sponge 30 is disposed within target site 70.The distal end of vacuum line 40 is attached to sponge 30 and extends proximally into GI tract 80 and out of the body. Patch 50 seals an opening between target site 70 and GI tract 80. Overtube 60 coaxially surrounds a portion of vacuum line 40. The distal end of overtube 60 abuts a proximal side of patch 50 via a connection region 56, such that overtube 60 is not connected to patch 50. Alternatively, connection region 56 may include an adhesive, a perforation, a snap-fit connection, a suture, or other connection mechanism to connect patch 50 to overtube 60. In use, a twisting or turning motion of overtube 60 about a longitudinal axis of overtube 60 may overcome the connection force between patch 50 and overtube 60 at connection region 56, allowing overtube 60 to be disconnected from patch 50 and removed from the body.

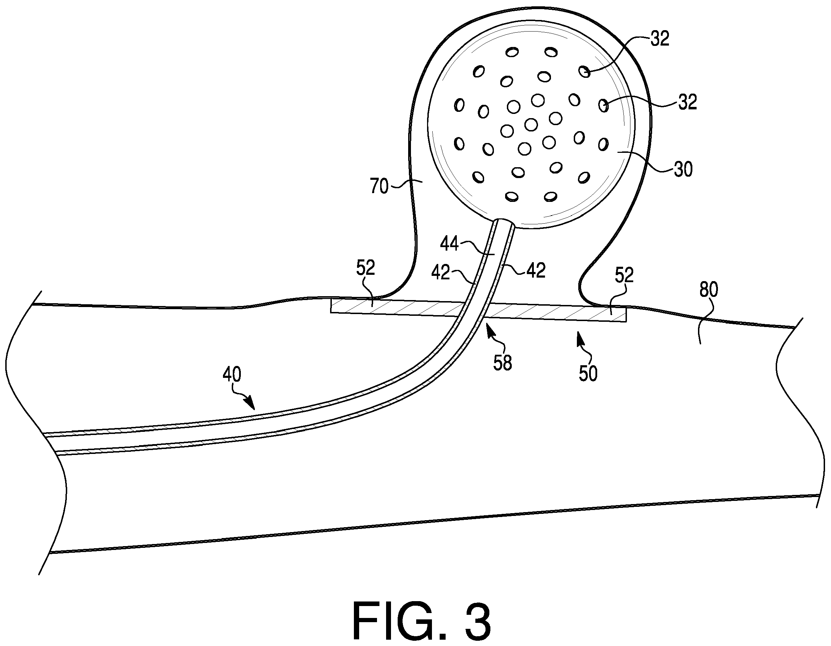

An example of patch 50 sealing target site 70 from GI tract 80 is shown in FIG. 3. As discussed herein, patch 50 may interact with a fluid in the body, e.g., fluid in GI tract 80, and may become adhesive such that body 52 of patch 50 may adhere to the tissue in GI tract 80 surrounding target site 70. Further, according to an example, the outer diameter of lumen 54 (FIG. 1) of patch 50 may be approximately equal to the diameter of the outer surface of outer wall 42 such that patch 50 is fluidly sealed with vacuum tube 40. In this manner, a fluid seal may be created between target site 70 and GI tract 80. Such a seal can inhibit or prevent material within GI tract 80 (e.g., fecal matter) from entering target site 70 and, for example, passing through a post-surgical leak and into another body organ. Since patch 50 is pushed along vacuum tube 40, vacuum tube 40 acts as a guide to aid patch 50 to be deployed at target site 70.

A method of using system 10 will now be described. Sponge 30 may be introduced through an orifice (e.g., a natural body orifice) and advanced to target site 70 using a catheter or other known mechanism. Once sponge 30 is disposed at target site 70, a proximal end of vacuum tube 40 is placed into lumen 54 of patch 50. The proximal end of vacuum tube 40 is subsequently placed into a distal end of central lumen 64 of overtube 60. Overtube 60 is pushed distally, causing patch 50 to move distally relative to vacuum tube 40. As patch 50 approaches target site 70, the user may urge or push patch 50 against the tissue surrounding target site 70. According to examples, the fluid of GI tract 80 may activate an adhesive on patch 50 to adhere patch 50 to the tissue. Alternatively, fluid may be introduced to patch 50 via a tool or an instrument to activate the adhesive of patch 50. After patch 50 is attached to the tissue surrounding target site 70, overtube 60 may be moved proximally and removed from the body via the orifice. As discussed herein, overtube 60 may be connected to patch 50 via an adhesive, snap fit, or the like. In this example, overtube 60 may be rotated about the longitudinal axis to overcome a connection force between overtube 60 and patch 50, thereby disconnecting overtube 60 from patch 50. Once overtube 60 has been removed from the body, vacuum line 40 may be attached to a vacuum source and a negative pressure of approximately 125 mm Hg, or approximately 2.5, may be supplied to sponge 30.

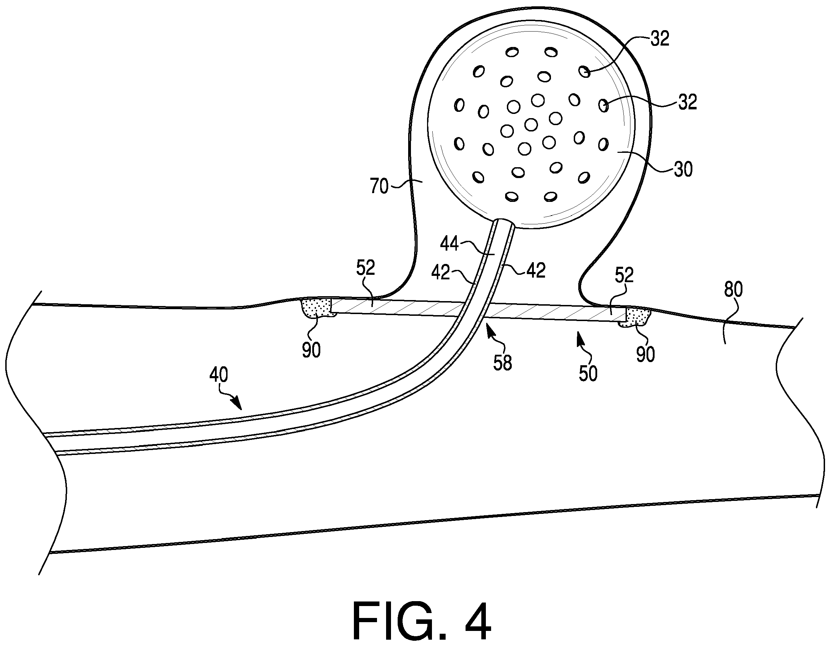

FIG. 4 illustrates an additional or alternative mechanism for sealing patch 50 to the tissue of GI tract 80. A fluid 90 may be sprayed along an intersection of patch 50 and the wall of GI tract 80, about the perimeter of patch 50. Fluid 90 may include a liquid-based Chitosan or other material suitable for sealing patch 50 to tissue on the wall of GI tract 80, including any suitable material mentioned in this disclosure. Additionally, or alternatively, sealing patch 50 may include modified versions of Chitosan such as a thiolated, a catechol, or an aldehyde of Chitosan, acrylated polyurethanes, methylmethacrylates, gelatin methacryloyl (GelMA), polyethylene glycol (PEG), or the like. Fluid 90 may further include any liquid, gel, aerosolized material, or the like. Fluid 90 may fill spaces between patch 50 and the wall of GI tract 80, which may decrease the possibility that fluid or other materials will travel from GI tract 80 into target site 70. Furthermore, fluid 90 may interact with and may seal to both patch 50 and the tissue of the wall of GI tract 80, which may increase the sealing strength between patch 50 and GI tract 80. This increased sealing strength may prevent patch 50 from becoming detached prematurely from the tissue when covering target site 70 due to, e.g., material or other debris traveling within GI tract 80. Fluid 90 may be introduced to a target area, e.g., at the intersection of patch 50 and the wall of GI tract 80, via a catheter or other delivery device. Fluid 90 may be in an aerosolized configuration and certain properties, e.g., an adhesive property, of fluid 90 may be activated when fluid 90 is contacted by a liquid. For example, moisture or liquid of GI tract 80 may activate fluid 90. Alternatively, or additionally, a liquid may be supplied by the catheter or other delivery device to activate fluid 90 after fluid 90 is supplied to GI tract 80.

EVAC device 20, including sponge 30 and vacuum tube 40, and patch 50 in FIG. 4 may be deployed to target site 70 in a similar manner discussed with reference to FIGS. 2 and 3. After positioning patch 50 at target site 70, fluid 90 may be deployed from a distal end of a catheter or other similar deployment device. A liquid or other activation agent may also be supplied to fluid 80. After deployment of fluid 90 and any activation agent, the catheter or other deployment device may be removed via the body orifice.

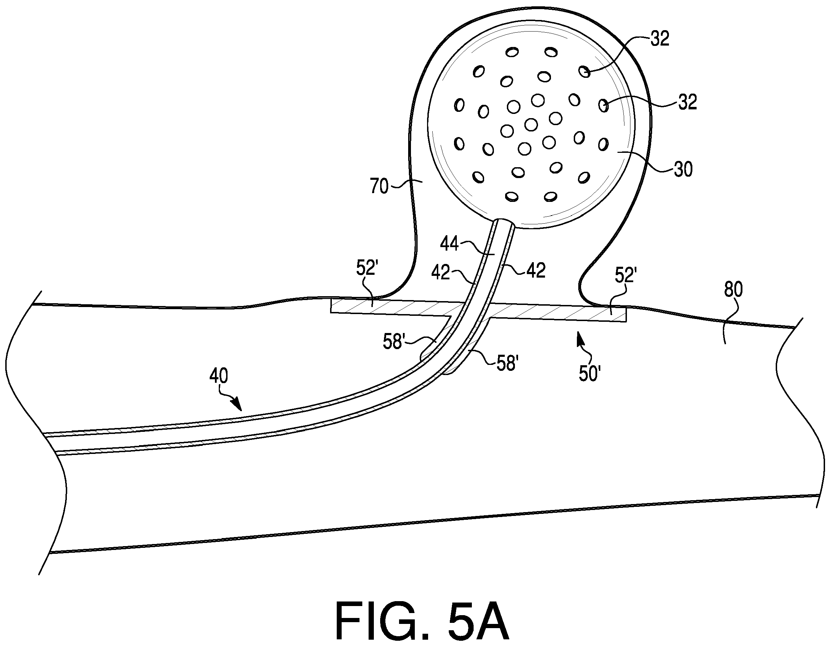

FIG. 5A illustrates another example of an EVAC system 10. EVAC system 10 of FIG. 5A includes similar components as shown in FIG. 1. A patch 50' may be similar to patch 50, and may further include a tube 58' (e.g., a patch tube) connected to and extending from a proximal surface of patch 50'. An inner lumen of patch 58' may be an extension of a lumen of patch 50' (e.g., lumen 54 of patch 50 shown in FIG. 1). When deployed in the body, tube 58' may extend proximally from patch 50' and may provide an additional seal around vacuum tube 40. In some examples, tube 58' may taper from patch 50' in the proximal direction. A diameter of the lumen defined by tube 58' may be approximately equal to the lumen of patch 50'. In some examples, the diameter of central lumen 64 of overtube 60 may be less than an outer diameter of tube 58' such that, during deployment, the distal end of overtube 60 may contact a proximal end of tube 58'. Alternatively, the diameter of central lumen 64 may be greater than the outer diameter of tube 58', such that the distal end of overtube 60 may contact a portion of patch 50' other than tube 58' during deployment, similar to the deployment of patch 50 described herein. It will be understood that EVAC system 10 may be deployed in any manner described herein. Tube 58' may also be flexible to bend and thereby conform to a shape of vacuum tube 40.

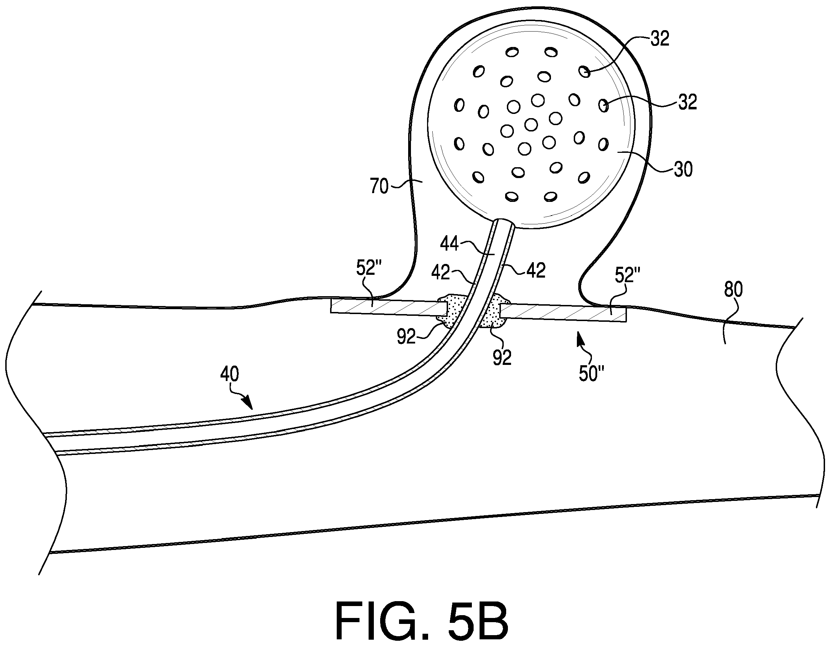

FIG. 5B illustrates another example of an EVAC system 10. EVAC system 10 of FIG. 5B includes similar components as shown in FIG. 1. A patch 50" may be similar to patch 50, and may include a lumen (similar to lumen 54 in FIG. 1) having a diameter greater than the outer diameter of vacuum tube 40. In this example, patch 50" may slide more freely along vacuum tube 40 during deployment and friction forces between a wall that defines the lumen of patch 50" and vacuum tube 40 during deployment may be reduced. Reducing these friction forces may reduce shearing and/or strain forces on vacuum tube 40 during deployment of patch 50", which may prevent or reduce deterioration of vacuum tube 40.

When deployed, a space or a gap may be created between patch 50" and vacuum tube 40, such that target site 70 is not completely sealed from GI tract 80. A seal 92 may be disposed in the space created between vacuum tube 40 and the wall defining the lumen of patch 50" to seal target site 70 from GI tract 80. For example, seal 92 may be similar to fluid 90 and may be, e.g., Chitosan or other material suitable for creating a seal between vacuum tube 40 and patch 50". Seal 92 may be deployed in a similar manner as fluid 90, e.g., via a catheter or the like. Alternatively, seal 92 may be a silicone seal (or other suitable material) placed between the wall defining the lumen of patch 50" and tube 40, and may be deployed using a catheter having an end effector capable of grasping seal 92 or other similar device.

Sponge 30 and patch 50" may be deployed in a manner similar to that described with respect to FIGS. 2 and 3. For example, after positioning sponge 30 at target site 70, patch 50" may be advanced along vacuum tube 40 to seal target site 70 from GI tract 80 using overtube 60. Once patch 50" is positioned at target site 70, seal 92 may be deployed into lumen 54" via a catheter, an end effector, or other deployment device. In some examples, fluid 90 shown in FIG. 4 may also be deployed at the intersection of patch 50" and the inner wall of GI tract 80 to further seal target site 70 from GI tract 80. After deployment, overtube 60 and any other deployment mechanisms may be removed from the body.

FIGS. 5C and 5D illustrate yet another example of a patch 50‴ according to an example. Sponge 30 is attached to the distal end of vacuum tube 40, similar to sponge 30' and vacuum tube 40 shown in FIG. 1. Patch 50‴ may be fixed to vacuum tube 40 proximal of sponge 30. According to an example, a body 52‴ of patch 50‴ may include a plurality of recesses 52a facing sponge 30'. Recesses 52a may be connected to one or more channels 52b defined by outer walls of patch 50"'. As shown in FIG. 5D, channels 52b may be connected by one or more openings 44a in vacuum tube 40, such that lumen 44 of vacuum tube 40 may be fluidly connected to recesses 52a. In this example, a negative pressure supplied to sponge 30' may also be supplied to recesses 52a via channels 52b. The vacuum supplied to recesses 52a may cause patch 50‴ to attach to the tissue of the sidewall of GI tract 80 surrounding the opening to target site 70, thereby sealing target site 70 from GI tract 80. Recesses 52a may be in any pattern and/or randomly spaced about the surface of 50‴ facing sponge 30'. In an example, some recesses 52a may overlap the opening between target site 70 and GI tract 80 and may provide additional paths for fluids and other materials to be removed from target site 70. Alternatively, or additionally, a second vacuum tube may be attached to patch 50‴. In this example, tube 40 does not require openings 44a, and vacuum pressure supplied lumen 44 is independently controlled of the vacuum pressure supplied to channels 52b and recesses 52a. The additional vacuum tube may allow patch 50‴ to be positioned and attached to the tissue of the sidewall of GI tact 80 and seal treatment site 70 from GI tract 80 independently of and, in some instances, before removing fluid or materials from treatment site 70. While patch 50‴ is fixedly attached to vacuum tube 40 in FIG. 5C, patch 50‴ may be placed over vacuum tube 40 and advanced to a target site in any manner described herein. For example, if patch 50‴ is attached to the second vacuum tube, separate from vacuum tube 40, patch 50‴ may be advanced along vacuum tube 40 and may be moved relative to vacuum tube 40 and independently of sponge 30'. Sponge 30' and patch 50‴ may be deployed to target site 70 and may remove debris and other material from target site 70 in any manner described herein. Patch 50‴ may be any material described herein suitable for sealing target site 70 from GI tract 80, e.g., Chitosan.

It will be understood that any of the patches described herein may be used alone or in combination with one or more sponges described herein. It will also be understood that features of any of the patches described herein may be used with any other patches described herein.

While different medical systems have been described, it will be understood that the particular arrangements of elements in these systems are not limited. Moreover, a size, a shape, and/or the materials of the sealing devices in the EVAC system are not limited. As described herein, there are included patches or sealing devices for sealing a sponge and a target site from a body lumen. For example, performing various medical procedures may be improved by ensuring a proper seal between the target site and any debris or materials in a body lumen. This seal may prevent these materials (e.g., fecal matter) from entering the target site and passing into another body organ through, e.g., a post-surgical leak, and causing infections or other medical issues.

It will be apparent to those skilled in the art that various modifications and variations can be made to the disclosed device without departing from the scope of the invention as defined by the following claims.

## Claims

1. A medical system (10) comprising:
a porous body (30, 30') connected to a distal end of a vacuum tube (40);
a patch (50, 50', 50", 50‴) defining a lumen (54) therethrough for accommodating the vacuum tube (40),
an overtube (60) configured to contact the patch (50, 50', 50", 50‴) during a deployment of the patch (50, 50', 50", 50‴) and configured to be releasably attached to the patch (50, 50', 50", 50‴) during the deployment of the patch (50, 50', 50", 50‴),
wherein the patch (50, 50', 50", 50‴) is configured to fluidly seal the porous body (30, 30') from a body lumen when the patch is deployed in a subject and the overtube (60) is configured to be disconnected from the patch (50, 50', 50", 50‴) when the patch is deployed in the subject.

2. The medical system (10) of claim 1, wherein a diameter of the lumen (64) of the patch (50, 50', 50", 50‴) is approximately equal to an outer diameter of the vacuum tube (40), and the patch (50, 50', 50", 50‴) is slidable along an exterior of the vacuum tube (40).

3. The medical system (10) of claims 1 or 2, wherein the patch (50') includes a patch tube (58') extending from a first surface of the patch (50'), wherein the patch tube (58') defines a tube lumen in fluid communication with the lumen of the patch, and wherein the patch tube (58') is configured to seal around a circumference of the vacuum tube (40) when the patch (50') is deployed in the subject.

4. The medical system (10) of any of the preceding claims, wherein the patch (50, 50', 50", 50‴) includes Chitosan.

5. The medical system (10) of any of the preceding claims, further comprising a sealing fluid configured to be applied to an intersection between the patch (50, 50', 50", 50‴) and a tissue of the subject.

6. The medical system (10) of claim 1, wherein a diameter of the lumen (54) of the patch (50, 50', 50", 50‴) is greater than an outer diameter of the vacuum tube (40), and wherein a gap is formed between the patch (50, 50', 50", 50‴) and the vacuum tube (40) when the patch (50, 50', 50", 50"') and the vacuum tube (40) are deployed in the body.

7. The medical system (10) of claim 6, wherein a sealing fluid is configured to be supplied to the gap to seal the body lumen (40) from the porous body (30).

8. The medical system (10) of claim 1, wherein the patch (50‴) defines a plurality of recesses (52a) on a surface of the patch (50‴) facing the porous body (30'), and wherein at least one recess of the plurality of recesses (52a) is configured to overlap a tissue of the body lumen in the deployed configuration.

9. The medical system (10) of claim 8, wherein the plurality of recesses (52a) are in fluid communication with a lumen (44) of the vacuum tube (40).

10. The medical system (10) of claim 9, wherein the plurality of recesses (52a) communicate with a lumen (44) of the vacuum tube (40) via one or more channels (52b) in the patch (50‴).

11. The medical system (10) of any of the preceding claims, wherein the porous body (30, 30') include a sponge.

12. The medical system (10) of any of the preceding claims, wherein the porous body (30, 30') includes a plurality of holes, pores, or channels (32, 32') permitting fluid outside of the porous body (30, 30') to flow through the porous body (30, 30') and into a lumen of the vacuum tube (40).

13. The medical system (10) of any of the preceding claims, further comprising a vacuum device configured to be attached to a proximal end of the vacuum tube (40) and configured to supply a negative pressure to the porous body (30, 30').

## Patentansprüche

1. Medizinisches System (10) aufweisend:
einen porösen Körper (30, 30'), der mit einem distalen Ende einer Vakuumröhre (40) verbunden ist;
ein Pflaster (50, 50', 50", 50‴), das ein hindurchgehendes Lumen (54) zur Aufnahme der Vakuumröhre (40) definiert,
eine Überröhre (60), die konfiguriert ist, das Pflaster (50, 50', 50", 50‴) während einer Anwendung des Pflasters (50, 50', 50", 50‴) zu kontaktieren, und konfiguriert ist, während der Anwendung des Pflasters (50, 50', 50", 50‴) lösbar mit dem Pflaster (50, 50', 50", 50‴) gekoppelt zu sein,
wobei das Pflaster (50, 50', 50", 50‴) konfiguriert ist, den porösen Körper (30, 30') gegen ein Körperlumen fluidundurchlässig abzudichten, wenn das Pflaster in einem Individuum angewendet wird, und die Überröhre (60) konfiguriert ist, von dem Pflaster (50, 50', 50", 50‴) getrennt zu werden, wenn das Pflaster in dem Individuum angewendet wird.

2. Medizinisches System (10) nach Anspruch 1, wobei ein Durchmesser des Lumen (54) des Pflasters (50, 50', 50", 50‴) ungefähr gleich einem Außendurchmesser der Vakuumröhre (40) ist und das Pflaster (50, 50', 50", 50‴) entlang einer Außenseite der Vakuumröhre (40) gleitbar ist.

3. Medizinisches System (10) nach Anspruch 1 oder 2, wobei das Pflaster (50') eine Pflasterröhre (58') aufweist, die sich von einer ersten Oberfläche des Pflasters (50') erstreckt, wobei die Pflasterröhre (58') ein Röhrenlumen in Fluidkommunikation mit dem Lumen des Pflasters definiert, und wobei die Pflasterröhre (58') konfiguriert ist, um einen Umfang der Vakuumröhre (40) herum abzudichten, wenn das Pflaster (50') in dem Individuum angewendet wird.

4. Medizinisches System (10) nach einem der vorstehenden Ansprüche, wobei das Pflaster (50, 50', 50", 50‴) Chitosan aufweist.

5. Medizinisches System (10) nach einem der vorstehenden Ansprüche, ferner aufweisend ein Dichtungsfluid, das konfiguriert ist, auf eine Grenzfläche zwischen dem Pflaster (50, 50', 50', 50‴) und einem Gewebe des Individuums aufgetragen zu werden.

6. Medizinisches System (10) nach Anspruch 1, wobei ein Durchmesser des Lumen (54) des Pflasters (50, 50', 50", 50‴) größer ist als ein Außendurchmesser der Vakuumröhre (40), und wobei ein Spalt zwischen dem Pflaster (50, 50', 50", 50‴) und der Vakuumröhre (40) gebildet ist, wenn das Pflaster (50, 50', 50", 50‴) und die Vakuumröhre (40) in dem Körper angewendet werden.

7. Medizinisches System (10) nach Anspruch 6, wobei ein Dichtungsfluid konfiguriert ist, dem Spalt zugeführt zu werden, um das Körperlumen gegen den porösen Körper (30) abzudichten.

8. Medizinisches System (10) nach Anspruch 1, wobei das Pflaster (50‴) mehrere Aussparungen (52a) an einer dem porösen Körper (30') zugewandten Oberfläche des Pflasters (50‴) definiert, und wobei mindestens eine Aussparung der mehreren Aussparungen (52a) konfiguriert ist, in der angewendeten Konfiguration ein Gewebe des Körperlumen zu überlappen.

9. Medizinisches System (10) nach Anspruch 8, wobei die mehreren Aussparungen (52a) in Fluidkommunikation mit einem Lumen (44) der Vakuumröhre (40) sind.

10. Medizinisches System (10) nach Anspruch 9, wobei die mehreren Aussparungen (52a) via einen oder mehrere Kanäle (52b) in dem Pflaster (50‴) mit einem Lumen (44) der Vakuumröhre (40) kommunizieren.

11. Medizinisches System (10) nach einem der vorstehenden Ansprüche, wobei der poröse Körper (30, 30') einen Schwamm aufweist.

12. Medizinisches System (10) nach einem der vorstehenden Ansprüche, wobei der poröse Körper (30, 30') mehrere Löcher, Poren oder Kanäle (32, 32') aufweist, die erlauben, dass Fluid von außerhalb des porösen Körpers (30, 30') durch den porösen Körper (30, 30') und in ein Lumen der Vakuumröhre (40) fließt.

13. Medizinisches System (10) nach einem der vorstehenden Ansprüche, ferner aufweisend eine Vakuumvorrichtung, die konfiguriert ist, an ein proximales Ende der Vakuumröhre (40) angeschlossen zu werden, und konfiguriert ist, dem porösen Körper (30, 30') einen Unterdruck zuzuführen.

## Revendications

1. Système médical (10) comprenant :
un corps poreux (30, 30') raccordé à une extrémité distale d'un tube à vide (40) ;
un timbre (50, 50', 50", 50‴) définissant une lumière (54) à travers ce dernier pour loger le tube à vide (40),
un surtube (60) configuré pour être en contact avec le timbre (50, 50', 50", 50‴) pendant un déploiement du timbre (50, 50', 50", 50‴) et configuré pour être fixé, de manière libérable, au timbre (50, 50', 50", 50"') pendant le déploiement du timbre (50, 50', 50", 50‴),
dans lequel le timbre (50, 50', 50", 50"') est configuré pour réaliser l'étanchéité, de manière fluidique, du corps poreux (30, 30') par rapport à une lumière corporelle lorsque le timbre est déployé dans un patient et le surtube (60) est configuré pour être déconnecté du timbre (50, 50', 50", 50‴) lorsque le timbre est déployé dans le patient.

2. Système médical (10) selon la revendication 1, dans lequel un diamètre de la lumière (64) du timbre (50, 50', 50", 50"') est approximativement égal à un diamètre externe du tube à vide (40) et le timbre (50, 50', 50", 50‴) peut glisser le long d'un extérieur du tube à vide (40).

3. Système médical (10) selon les revendications 1 ou 2, dans lequel le timbre (50') comprend un tube à timbre (58') s'étendant à partir d'une première surface du timbre (50'), dans lequel le tube à timbre (58') définit une lumière de tube en communication de fluide avec la lumière du timbre, et dans lequel le tube à timbre (58') est configuré pour réaliser l'étanchéité autour d'une circonférence du tube à vide (40) lorsque le timbre (50') est déployé dans le patient.

4. Système médical (10) selon l'une quelconque des revendications précédentes, dans lequel le timbre (50, 50', 50", 50‴) comprend de la chitosane.

5. Système médical (10) selon l'une quelconque des revendications précédentes, comprenant en outre un fluide d'étanchéité configuré pour être appliqué à une intersection entre le timbre (50, 50', 50", 50"') et un tissu du patient.

6. Système médical (10) selon la revendication 1, dans lequel un diamètre de la lumière (54) du timbre (50, 50', 50", 50"') est supérieur à un diamètre externe du tube à vide (40) et dans lequel un interstice est formé entre le timbre (50, 50', 50", 50‴) et le tube à vide (40) lorsque le timbre (50, 50', 50", 50‴) et le tube à vide (40) sont déployés dans le corps.

7. Système médical (10) selon la revendication 6, dans lequel un fluide d'étanchéité est configuré pour être amené à l'interstice pour réaliser l'étanchéité de la lumière corporelle par rapport au corps poreux (30).

8. Système médical (10) selon la revendication 1, dans lequel le timbre (50‴) définit une pluralité d'évidements (52a) sur une surface du timbre (50‴) faisant face au corps poreux (30'), et dans lequel au moins un évidement de la pluralité d'évidements (52a) est configuré pour recouvrir un tissu de la lumière corporelle dans la configuration déployée.

9. Système médical (10) selon la revendication 8, dans lequel la pluralité d'évidements (52a) est en communication de fluide avec une lumière (44) du tube à vide (40).

10. Système médical (10) selon la revendication 9, dans lequel la pluralité d'évidements (52a) communique avec une lumière (44) du tube à vide (40) via un ou plusieurs canaux (52b) dans le timbre (50‴).

11. Système médical (10) selon l'une quelconque des revendications précédentes, dans lequel le corps poreux (30, 30') comprend une éponge.

12. Système médical (10) selon l'une quelconque des revendications précédentes, dans lequel le corps poreux (30, 30') comprend une pluralité de trous, de pores ou de canaux (32, 32') permettant au fluide à l'extérieur du corps poreux (30, 30') de s'écouler par le corps poreux (30, 30') et dans une lumière du tube à vide (40).

13. Système médical (10) selon l'une quelconque des revendications précédentes, comprenant en outre un dispositif sous vide configuré pour être fixé à une extrémité proximale du tube à vide (40) et configuré pour fournir une pression négative au corps poreux (30, 30').
